# EUROPÄISCHE PATENTANMELDUNG

(11) **EP 1 985 692 A1**
(43) Veröffentlichungstag der Anmeldung: **29.10.2008**
(21) Anmeldenummer: 07008219.3
(22) Anmeldetag: 23.04.2007
(51) Int. Cl.: C12M 1/08

(54) **Biogasanlage zur Herstellung von Biogas in einem einstufigen Durchflussverfahren**

(71) Anmelder: UTS Biogastechnik GmbH, 84419 Obertaufkirchen (DE)
(72) Erfinder: Bürger, Adam, 83527 Haag/Obb. (DE)
(74) Vertreter: Liebl, Thomas

(57) **Zusammenfassung**

Die Erfindung betrifft eine Biogasanlage zur Herstellung von Biogas durch anaerobe Nassvergärung eines Biomasse enthaltenden Substrats in einem einstufigen Durchflussverfahren, mit wenigstens einem Fermenterbehälter (1) mit einer Rühreinrichtung (19, 20) und mit einer Zudosiereinrichtung für flüssige und feste Substratbestandteile. Erfindungsgemäß besteht der wenigstens eine Fermenterbehälter (1) aus zwei parallel verlaufenden, langgestreckten und gegensinnig durchströmten Längskanälen (10, 11), wobei die jeweils benachbarten Kanalenden der Längskanäle (10, 11) durch Kanalumlenkbögen (13, 14) miteinander verbunden sind, dergestalt, dass eine ringförmig langgestreckt umlaufende Endlos-Kanalstruktur als Langfermenter gebildet ist.

## Beschreibung

Die Erfindung betrifft eine Biogasanlage zur Herstellung von Biogas durch anaerobe Nassvergärung eines Biomasse enthaltenden Substrats in einem einstufigen Durchflussverfahren nach dem Oberbegriff des Anspruchs 1.

Die Biogasgewinnung zur Energieerzeugung gewinnt vorrangig im landwirtschaftlichen Bereich zunehmend an Bedeutung. Die Gründe dafür sind im wesentlichen der Erschließung neuer Einkommensquellen in der Landwirtschaft durch eine staatliche geförderte regenerative und umweltverträgliche Energiegewinnung. Die dafür bekannten Biogasanlagen sind meist für einen kontinuierlichen einstufigen Betrieb ausgelegt mit einem Fermenterbehälter, in dem in einem anaeroben Nassvergärungsprozess zur Gewinnung von Biogas Biomassen vergoren werden. Je nach den Gegebenheiten sind einem solchen Fermenter ein Nachfermenter und ein Endlager sowie separate Gasspeicher für das erzeugte Biogas nachgeordnet. Zur Beschickung des Fermenterbehälters mit vergärbaren Stoffen ist diesem eine meist automatisierte Zudosiereinrichtung vorgeschaltet, wobei für flüssige Substratbestandteile Pumpen und für feste Biomassen Feststoff-Zudosiereinrichtungen ggf. mit Wägeeinrichtungen und Befüllschnecken oder Förderbändern verwendet werden. Zudem sind in den Behältern und Fermentern Rühreinrichtungen für eine gleichmäßige Verteilung der Substratbestandteile angebracht (EP 1 251 165 A1).

Ein Problem bei solchen Fermentationsprozessen ist in allgemein bekannter Weise, dass die Biomasse insbesondere Biomassenfeststoffe in der Fermenterflüssigkeit regelmäßig nicht gleichmäßig verteilt sind, da sie z. B. aufschwimmen und sich im Bereich der Flüssigkeitsoberfläche ansammeln, wenn das spezifische Gewicht der Biomasse geringer ist als dasjenige der Fermenterflüssigkeit, z. B. von Wasser. Andererseits kann aber das Problem bestehen, dass Biomasse bei einem höheren spezifischen Gewicht auf den Fermenterbehälterboden absinkt und sich dort Sinkschichten ansammeln. Auch eine derartige Ansammlung von Biomasse bewirkt eine ungleichmäßige Biomasseverteilung in der Fermenterflüssigkeit, was sich insgesamt nachteilig auf den Wirkungsgrad des Fermentationsprozesses und damit nachteilig auf den Wirkungsgrad der Biogasherstellung auswirkt.

Für kontinuierlich in einem einstufigen Durchflussverfahren arbeitende Biogasanlagen sind bisher zylindrisch aufgebaute Fermenterbehälter in grundsätzlich zwei Anordnungen verwendet worden:

Beispielsweise ist ein Fermenter einer Biogasanlage mit einer Rühreinrichtung für eine Durchmischung bekannt (DE 199 28 212 A1), bei der der Fermenterbehälter aus einem zylinderrohrförmigen liegenden Tank mit einer horizontalen Zylinderachse und mit einer zentral horizontal liegenden Rührwelle besteht, an der axial und radial zueinander versetzt eine Mehrzahl von Rührpaddeln angeordnet sind. Ein solcher liegender Tank mit einer Paddelrühreinrichtung kann mit vertretbaren Mitteln nicht beliebig groß gestaltet werden, so dass entsprechende Fermenterbehälter zweckmäßig nur in begrenzten Größenordnungen projektiert werden. Nachteilig sind Reparatur- und Wartungsarbeiten an solchen Paddelrühreinrichtungen nur mit erheblichem Aufwand und bei entleertem Fermenterbehälter möglich. Eine Anordnung mehrerer solcher liegender Zylindertanks zur Herstellung einer großen Biogasanlage erfordert ungünstig viel Platz, da zwischen den liegenden Zylindertanks viel ungenützter Platz verbleibt.

Bei einer anderen Ausführungsform, werden zylindrische Fermenterbehälter mit vertikaler Zylinderachse meist als Hochbehälter oder teilweise im Erdboden versenkte Behälter aus Beton oder Stahl eingesetzt. (EP 1 130 084 B1; DE 200 08 186 U1), die in allgemein bekannter Weise durch eine Betondecke, eine Stahldecke oder durch ein ein- oder zweilagiges Foliendach, welches ggf. einen Gasspeicher bildet, gasdicht abgedeckt sind. Bei solchen zylindrischen Fermenterbehältern werden regelmäßig Rühreinrichtungen in der Art von höhenverstellbaren Tauchrührgeräten oder seitlich durch eine Behälterwand eingeführten Rührgeräten verwendet. Auch hier ergeben sich bei nebeneinander angeordneten zylindrischen Behältern, beispielsweise einem Hauptfermenter, einem Nachfermenter und einem Endlager eine unzureichende Grundflächenausnützung, durch rundungsbedingte schlecht nutzbare Freiräume zwischen den Behältern. Bei großen Biogasanlagen mit einer Mehrzahl von Fermenterbehältern wächst dieser Nachteil erheblich. Wenn zur Behebung eines solchen Nachteils die Behälterdurchmesser größer gewählt werden sollten, führt dies zu bautechnisch hohem Aufwand und zudem besteht das Problem, dass eine Einpassung solcher großer Zylinderbehälter mit großer Bodenflächenausdehnung in allen Richtungen in vorhandene Geländestrukturen und Grundstückseigentumsverhältnisse zu Schwierigkeiten führen kann. Rechteckige Fermenterbehälter, die einfach aneinander gereiht werden könnten, sind wegen rührtechnisch unzureichend erfassbarer Eckräume ungeeignet. Jedoch ist auch das Rühren und effektive Durchmischen des Substrats mit Rühreinrichtungen, beispielsweise mit Tauchrührgeräten in den vorstehenden zylindrischen Behältern grundsätzlich schwierig, da beispielsweise bei einer gleichmäßig umlaufenden Substratbewegung im Zylinderbehälter im Bereich der Zylinderachse praktisch keine Substratbewegung und Substratdurchmischung erfolgt. Um dem entgegenzuwirken ist es erforderlich, in aufwendiger Weise die Höhenstellungen und Winkelstellungen der Tauchrührgeräte regelmäßig zu verändern und den Rühr- und Durchmischungsvorgang mit relativ hohem Energieaufwand durchzuführen.

Aufgabe der Erfindung ist es demgegenüber eine Biogasanlage vorzuschlagen, bei der zur Vermeidung von Schwimm- und Sinkschichten eine effektivere und energiesparendere Rühreinrichtung verwendbar ist und zudem Projektierungsfreiräume hinsichtlich eines kompakten Aufbaus bei sparsamer Verwendung der erforderlichen Grundfläche möglich sind.

Diese Aufgabe wird mit den Merkmalen des Anspruchs 1 gelöst.

Gemäß Anspruch 1 besteht der wenigstens eine Fermenterbehälter aus zwei parallel verlaufenden, langgestreckten und gegensinnig durchströmten Längskanälen, wobei die jeweils benachbarten Kanalenden der Längskanäle durch Kanalumlenkbögen miteinander verbunden sind dergestalt, dass eine ringförmig langgestreckt umlaufende Endloskanalstruktur als Langfermenter gebildet ist.

Durch die umlaufende Endloskanalstruktur mit benachbarten gegenläufigen Längskanälen und Kanalumlenkbögen kann eine entsprechend umlaufende Substratströmung erzeugt werden, welche hinsichtlich der Durchmischung besonders effektiv für den Fermentationsprozess und damit für eine effektive Biogasausbeute ist. Da hier ersichtlich weitgehend laminare gleichmäßige umlaufende Strömungsverhältnisse vorliegen, ohne dass die Strömung stark durch Umlenkungen, ungleichmäßige Strömungsgeschwindigkeiten oder Turbulenzen wesentlich beeinflusst ist, ist der Energieaufwand zur Aufrechterhaltung einer solchen Strömung vergleichsweise gering. Die gesamte Energiebilanz der Biogasanlage unter Einbeziehung der Rührtechnik wird somit verbessert, ohne dass die Gefahr von ungünstigen Ausbildungen von Schwimm- oder Sinkschichten gegeben ist.

Durch die langgestreckte Ausgestaltung der Fermenterbehälter in der Art von Langfermentern können mehrere Fermenterbehältern mit ihren Langseiten eng aneinander gereiht werden, ohne dass schwierig nutzbare Zwischenräume entstehen. Zudem ergeben sich dadurch Projektierungsfreiräume zur Anpassung an Platzverhältnisse hinsichtlich von Bodenstrukturen und Grundbesitzverhältnissen.

Konkret wird mit Anspruch 2 vorgeschlagen, dass der Fermenterbehälter als langgestreckte Fermenterwanne mit endseitigen Wannenrundungen ausgeführt ist, wobei die langgestreckten gegensinnig durchströmten Längskanäle beidseitig einer in der Längsmitte der Wanne verlaufenden vertikalen Mittenwand gebildet sind. Zur Ausbildung der Kanalumlenkbögen hat die Mittenwand eine kürzere Länge im Vergleich zur Innenlänge der Fermenterwanne und weist jeweils einen Mittenwandlängsabstand zwischen den Mittenwandlängsenden zu der in Längsrichtung gesehenen Innenseite der Wannenrundung auf. Durch eine gemeinsame Wand der Längskanäle als Mittenwand einer Fermenterwanne ergibt sich ein sehr platzsparender und kompakter Gesamtaufbau, wobei auch die Kanalumlenkbögen bautechnisch einfach und kostengünstig im Gesamtbauwerk integrierbar sind.

Um die Strömungsumlenkung in den Kanalumlenkbögen zu begünstigen, wird mit Anspruch 3 vorgeschlagen, dass der Mittenwandlängsabstand zwischen den Mittenwandlängsenden und der jeweils zugeordneten in Längsrichtung gesehenen Innenseite der Wandrundung größer oder zumindest gleich der Quererstreckung eines Längskanals ist.

Ein strömungsmäßig effektiver, kostengünstiger Gesamtaufbau wird nach Anspruch 4 dadurch erreicht, dass die Längskanäle eine Bodenwand, äußere vertikale parallele Längsseitenwände sowie eine gemeinsame Mittenwand aufweisen, wodurch sich jeweils ein rechteckiger Kanalquerschnitt ergibt. Die endseitig anschließenden Kanalumlenkbögen sind dabei in einfacher Weise durch die Bodenwand und halbkreisförmig gebogene, äußere vertikale Bogenwände gebildet.

Um die vorstehenden positiven Effekte und Vorteile zu optimieren, soll nach Anspruch 5 die Länge des Längskanals mit den im wesentlichen gerade verlaufenden Kanalwänden wenigstens so lang wie seine Quererstreckung sein. Vorzugsweise soll diese Länge jedoch wenigstens zweimal und besonders bevorzugt wenigstens 2,5mal so lang sein. Die positiven Effekte treten bei langen Längskanalerstreckungen umso deutlicher auf. Dabei soll auch eine angemessene Quererstreckung vorgesehen werden, da sonst die Kanalumlenkbögen relativ eng werden und dadurch die Gleichmäßigkeit einer umlaufenden Substratströmung gestört werden könnte.

Konkret sind als vorgeschlagene Langfermenter sehr große Behälter bis 10.000 m³ und mehr bei Quererstreckungen von beispielsweise 10 bis 20 m, Längserstreckungen von 50 bis 100 m und Höhen von 10 m einfach möglich, wie sie bisher mit den bekannten Fermenterausführungen für Biogasanlagen in der Größe nicht verwendet wurden.

Auch längsseits aneinander gereihte kleinere Behältereinheiten können bauraumeffektiv und rührtechnisch gut beherrschbar zu Großanlagen kombiniert werden.

Gemäß Anspruch 6 können die Wände solcher Fermenterbehälter je nach den Gegebenheiten aus Beton und/oder Stahl und/oder Kunststoff, insbesondere aus zusammenfügbaren Fertigteilelementen hergestellt werden.

Im Vergleich zu zylindrischen Fermenterbehältern sind die gerade verlaufenden Außenwände der Längskanäle in Querrichtung durch das nach außen drängende Gewicht der Substratflüssigkeit statisch stärker beansprucht. Um mit den Außenwänden die statischen Erfordernisse erfüllen zu können und diese dennoch kostengünstig ohne zu große Wandstärken ausführen zu können wir mit Anspruch 7 vorgeschlagen, die Außenwände in Querrichtung durch äußere Stützbauten, beispielsweise Betonstützen abzustützen. Zusätzlich oder ggf. alternativ können auch gegenüberliegende Außenwände im oberen Bereich durch Zugklammern zusammengespannt werden.

Vorzugsweise können solche Zugklammern als begehbare und Technikelemente tragende Brückenstege ausgeführt werden, so dass diesen eine Doppelfunktion zukommt.

Längskanäle und Kanalumlenkbögen die durch eine Bodenwand und vertikale Kanalwände gebildet sind, müssen gasdicht abgedeckt werden. Dazu wird mit Anspruch 8 vorgeschlagen, eine solche Abdeckung durch eine Betondecke und/oder durch Stahlplatten und/oder durch ein Foliendach gasdicht auszuführen. Alle diese Dachaufbauten sind allgemein bekannt. Insbesondere sind auch mehrlagige Foliendächer bekannt, welche im Innenbereich als Gasspeicher verwendet sind. Die vorstehenden unterschiedlichen Arten von Abdeckungen können je nach den Gegebenheiten auch zur gasdichten Abdeckung von Teilkanalflächen kombiniert werden.

Konkret wird dazu in einer bevorzugten Ausführungsform nach Anspruch 9 vorgeschlagen, dass die Längskanäle durch ein einfach ausführbares giebeldachförmiges Foliendach und die halbkreisförmigen Kanalumlenkbögen jeweils durch eine Betondecke abgedeckt sind.

Auch bei der erfindungsgemäßen Fermenterbehälterausführung können nach Anspruch 10 in den Abdeckungen, insbesondere in den Betondecken- und/oder Foliendachbereichen Revisionsöffnungen, insbesondere Revisionsschächte und/oder Sichtfenster angeordnet werden. Zur Herstellung der beschriebenen Strömung werden nach Anspruch 11 vorzugsweise an den Längskanälen und/oder ggf. auch in den Kanalumlenkbögen wenigstens eine Rühreinrichtung angeordnet, mit der das Substrat im Fermenterbehälter in eine in der Endloskanalstruktur umlaufende Bewegung versetzbar ist. Gut geeignet sind nach Anspruch 12 Tauchrührgeräte mit einem elektrisch oder hydraulisch angetriebenen Motor, die vorzugsweise höhenverstellbar durch einen Revisionsschacht im Fermenterbehälter oder ggf. an einem Brückensteg angeordnet sind. Bei einer Anordnung solcher Tauchrührgeräte in den Längskanalbereichen können diese in Strömungsrichtung justiert werden, wodurch einerseits eine effektive Funktion erreicht wird und die bisher übliche Einrichtung für eine Winkelverstellung der Tauchrührgeräte entfallen kann. Grundsätzlich können jedoch auch hier an sich bekannte Rührgeräteanordnungen durch eine Fermenteraußenwand eingesetzt und verwendet werden.

Kompakte Anordnungen auch im Hinblick auf Kombinationen zu Großanlagen ergeben sich nach Anspruch 13 dadurch, dass wenigstens zwei Fermenterbehälter und/oder ein Endlagerbehälter mit gleichem Aufbau längsparallel nebeneinander angeordnet sind. Unter Einbeziehung eines befahrbaren Silos ist es zweckmäßig, auch eine Silowand längsparallel unmittelbar benachbart zu einer Fermenterbehälterlängswand anzuordnen. In einer Weiterbildung einer solchen Anordnung wird mit Anspruch 14 vorgesehen, dass zumindest einer der Fermenterbehälter oder ein Endlagerbehälter eine vergleichsweise geringere Längserstreckung aufweist und im dem dadurch geschaffenen Freiraum ein Technikgebäude angeordnet ist. In einem solchen Technikgebäude können beispielsweise Pumpen, Steuerungen, ein Blockheizkraftwerk, ein Gasspeicher oder andere Technikbauelemente der Biogasanlage angebracht sein. Insgesamt wird dadurch eine sehr kompakte Anlagengröße unter vorteilhaft geringem Verbrauch von Bodenfläche erreicht.

Anhand einer Zeichnung wird die Erfindung näher erläutert.

Es zeigen:
- Fig. 1: eine Draufsicht auf einen Fermenterbehälter (ohne Abdeckung),
- Fig. 2: eine Draufsicht und Schnitte durch eine erste Ausführungsform eines Fermenterbehälters ähnlich Fig. 1,
- Fig. 3: eine Draufsicht und Schnitte einer zweiten Ausführungsform eines Fermenterbehälters,
- Fig. 4: eine Draufsicht und einen Längsschnitt einer dritten Ausführungsform eines Fermenterbehälters,
- Fig. 5: eine schematische Draufsicht auf eine Anordnung einer Biogasanlage und,
- Fig. 6: eine schematische Draufsicht auf eine Anordnung einer Biogas-Großanlage.

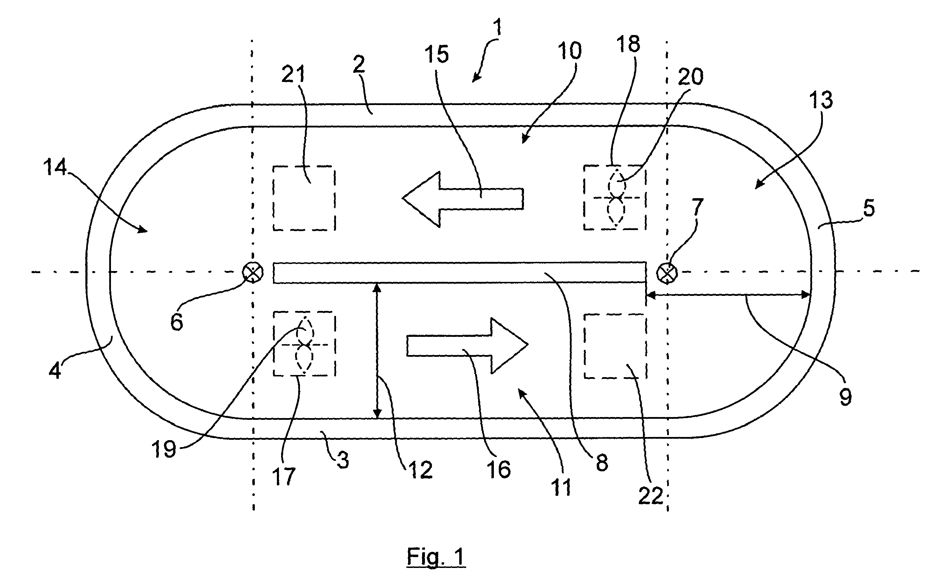

In Fig. 1 ist eine Draufsicht auf einen Fermenterbehälter 1 einer Biogasanlage gezeigt. An sich bekannte weitere Bauteile der Biogasanlage, insbesondere eine Zudosiereinrichtung, sowie die Abdeckung des Fermenterbehälters 1 sind wegen der besseren Übersichtlichkeit weggelassen. Der Fermenterbehälter 1 ist als langgestreckte Fermenterwanne ausgeführt mit jeweils gegenüberliegenden geraden vertikalen und parallelen Längsseitenwänden 2, 3, an die sich halbkreisförmig gebogene äußere vertikale Bogenwände 4, 5 als Wannenrundungen anschließen. Die halbkreisförmigen Bogenwände 4, 5 haben jeweils einen Kreismittelpunkt 6, 7, so dass die Längsseitenwände 2, 3 gerade im Bereich zwischen den durch die Kreismittelpunkte 6, 7 eingezeichneten strichpunktierten Querlinien verlaufen.

Weiter ist eine Mittenwand 8 im Fermenterbehälter 1 errichtet, die vertikal in der Längsmitte verläuft und deren Mittenwandlängsenden in einem Mittenwandlängsabstand entsprechend Pfeil 9 von der in Längsrichtung gesehenen Innenseite der jeweiligen Bogenwände 4, 5 liegen.

Durch den wannenförmigen Behälter 1 in Verbindung mit der Mittenwand 8 werden zwei langgestreckte gegensinnig durchströmbare Längskanäle 10, 11 ausgebildet. Die Quererstreckung entsprechend Pfeil 12 der Längskanäle 10 und 11 ist jeweils geringer als die Mittenwandlängsabstände 9, wodurch jeweils Kanalumlenkbögen 13, 14 an den jeweiligen Endseiten der Längskanäle 10, 11 ausgebildet sind. Dadurch sind im Längskanal 10 eine Substratströmung entsprechend dem Strömungspfeil 15 und im anderen Längskanal 11 eine gegensinnige Strömung entsprechend dem Strömungspfeil 16 herstellbar, welche unter Einbeziehung der Kanalumlenkbögen 13, 14 zu einer endlos umlaufenden Strömung führt. Der Rückversatz der Mittenwandenden (Pfeil 9) und damit der größere Querdurchmesser in den Bereichen der Kanalumlenkbögen 13, 14 im Vergleich zur Quererstreckung (Pfeil 12) in den Längskanälen 10, 11 begünstigt die Strömungsumlenkung von einem Längskanal in den anderen.

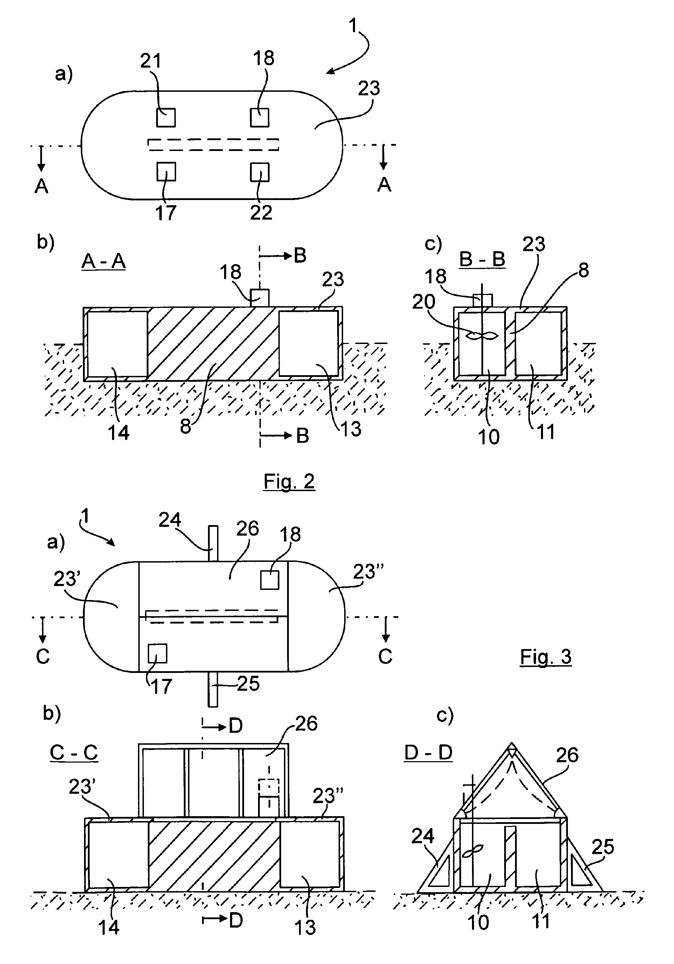

Schematisch und strichliert sind die Lagen von Serviceschächten 17, 18 in einer möglichen Betonabdeckung (siehe Fig. 2) eingezeichnet, in denen an sich bekannte höhenverstellbare Tauchrührgeräte 19, 20 zur Erzeugung der Substratströmung aufgenommen sind. Vorteilhaft sind die Tauchrührgeräte hier strömungseffektiv im Bereich der Längsströmung in den Längskanälen 10, 11 angeordnet. Zudem ist die Möglichkeit von Sichtfenstern 21, 22 in einer Beton- oder ggf. Stahlabdeckung angedeutet.

Fig. 2 zeigt weitere Einzelheiten der ersten Ausführungsform entsprechend Fig. 1. Dabei entspricht Fig. 2a der Fig. 1, wobei hier eine Abdeckung als Betondecke 23 angebracht ist, wie dies insbesondere im Längsschnitt Fig. 2b und im Querschnitt Fig. 2c ersichtlich ist. Die weiteren Fig. 1 entsprechenden Bauteile sind mit gleichen Bezugszeichen bezeichnet. Aus den Schnittdarstellungen ist zu ersehen, dass die Mittenwand 8 nach oben bis zur Betondecke 23 durchgeht und diese mitträgt. Grundsätzlich könnte die Mittenwand 8 oben auch mit einem Freiraum zur Betondecke 3 enden.

In einer zweiten Ausführungsform nach Fig. 3 ist der Grundaufbau des Fermenterbehälters 1 gleich entsprechend Fig. 1 und 2, so dass auch in Fig. 3 für gleiche Bauteile gleiche Bezugszeichen verwendet sind. In Fig. 2 ist zur Abstützung von Seitenkräften der Fermenterbehälter teilweise im Boden versenkt. In der Ausführungsform nach Fig. 3 sind dagegen bei einem Hochbehälter die besonders statisch nach außen belasteten Längsseitenwände 2, 3 jeweils durch Stützbauten 24, 25 abgestützt. Zudem sind bei der Ausführungsform nach Fig. 3 lediglich die Bereiche der Kanalumlenkbögen 13, 14 durch halbkreisförmige Betondeckenteile 23' und 23" abgedeckt, während der Bereich der Längskanäle 10, 11 durch ein giebeldachförmiges Foliendach 26 abgedeckt ist, welches, wie in Fig. 3c strichliert angedeutet, in an sich bekannter Weise auch zweilagig ausgeführt sein kann.

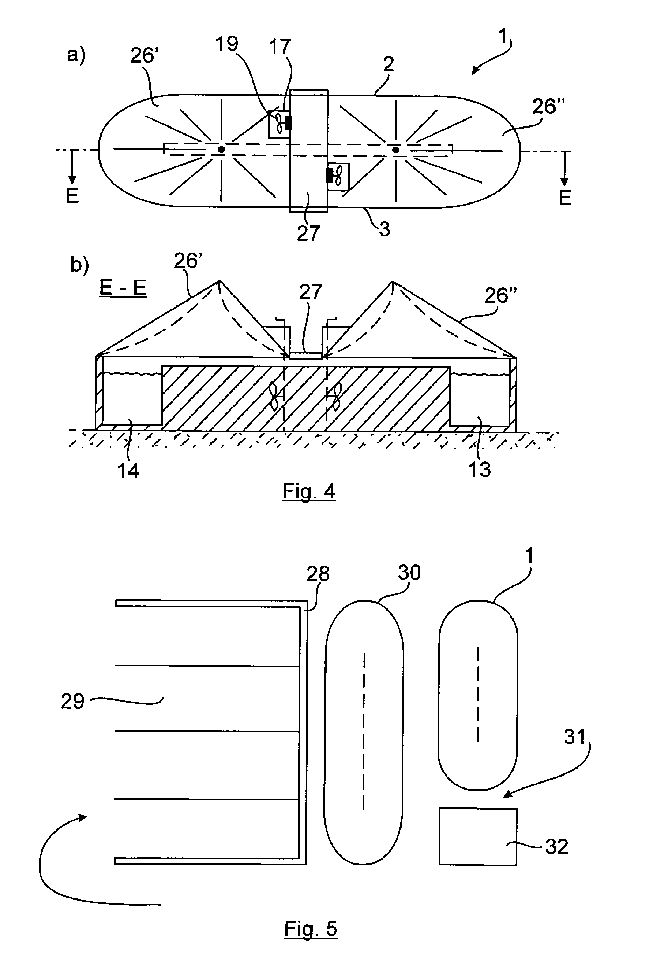

Die dritte Ausführungsform nach Fig. 4 entspricht weitgehend der zweiten Ausführungsform nach Fig. 3, so dass auch hierfür gleich Bauteile gleich Bezugszeichen verwendet sind. Der Unterschied besteht hier darin, dass die beiden Längsseitenwände 2, 3 durch einen Brückensteg 27 verbunden sind, der einerseits zur Stabilisierung die Funktion einer Zugklammer haben kann und andererseits für Wartungszwecke begehbar ist und eine Tragfunktion beispielsweise für Serviceschächte 17 und Tauchrührgeräte 19 haben kann. Als Abdeckung sind hier zwei Foliendachbereiche 26' und 26" verwendet, die sich ausgehend vom Brückensteg 27 auch über die Bereiche der Kanalumlenkbögen 13, 14 erstrecken.

Aus Fig. 5 ist die Möglichkeit eines sehr kompakten Aufbaus einer Biogasanlage in einer schematischen Draufsicht ersichtlich. Dabei ist parallel zu einer Silowand 28 eines Fahrsilos 29 ein Endlagerbehälter 30 mit dem erfindungsgemäßen Aufbau und einer Länge entsprechend der Silowand 28 angeordnet. Längsparallel zu diesem ist ein etwas kürzerer Fermenterbehälter 1 angebracht, so dass dadurch in einem Freiraum 31 Platz für ein Technikgebäude 32 geschaffen ist. Insgesamt wird somit hier eine etwa rechteckige Grundfläche optimal ausgenützt, ohne dass nicht oder nur schlecht nutzbare Freiräume verbleiben.

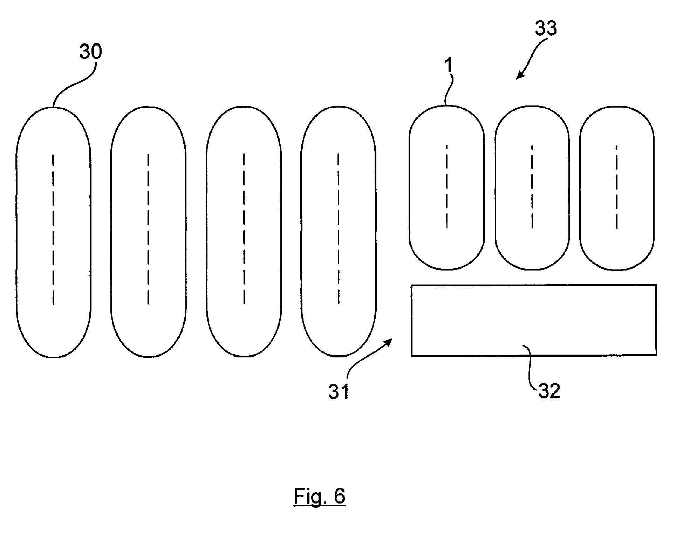

In Fig. 6 ist der Aufbau nach Fig. 5 weiter zu einer Anordnung einer Biogas-Großanlage 33 weitergeführt, wobei hier vier längere Endlagerbehälter 30 mit drei kürzeren Fermenterbehältern 1 in einer parallelen Anreihung kombiniert sind, so dass auch hier ein Freiraum 31 für ein Technikgebäude 32 verbleibt und auch hier eine optimale Grundflächenausnützung vorliegt.

## Patentansprüche

1. Biogasanlage zur Herstellung von Biogas durch anaerobe Nassvergärung eines Biomasse enthaltenden Substrats in einem einstufigen Durchflussverfahren,
mit wenigstens einem Fermenterbehälter (1) mit einer Rühreinrichtung (19, 20) und
mit einer Zudosiereinrichtung für flüssige und feste Substratbestandteile,
**dadurch gekennzeichnet,**
**dass** der wenigstens eine Fermenterbehälter (1) aus zwei parallel verlaufenden, langgestreckten und gegensinnig durchströmten Längskanälen (10, 11) besteht, wobei die jeweils benachbarten Kanalenden der Längskanäle (10, 11) durch Kanalumlenkbögen (13, 14) miteinander verbunden sind, dergestalt, dass eine ringförmig langgestreckt umlaufende Endlos-Kanalstruktur als Langfermenter gebildet ist.

2. Biogasanlage nach Anspruch 1, **dadurch gekennzeichnet, dass** der Fermenterbehälter (1) als langgestreckte Fermenterwanne mit endseitigen Wannenrundungen (4, 5) ausgeführt ist,
dass die langgestreckten, gegensinnig durchströmten Längskanäle (10, 11) beidseitig einer in der Längsmitte der Wanne verlaufende vertikalen Mittenwand (8) gebildet sind, und
dass die Mittenwand (8) eine kürzere Länge im Vergleich zur Innenlänge der Fermenterwanne aufweist, dergestalt, dass zur Ausbildung der Kanalumlenkbögen (13, 14) die Mittenwandlängsenden jeweils in einem Mittenwandlängsabstand (9) von der in Längsrichtung gesehenen jeweiligen Innenseite der Wannenrundung (4, 5) liegen.

3. Biogasanlage nach Anspruch 2, **dadurch gekennzeichnet, dass** jeweils der Mittenwandlängsabstand (9) größer oder zumindest gleich der Quererstreckung (12) eines Längskanals (10, 11) ist.

4. Biogasanlage nach Anspruch 1 oder 2, **dadurch gekennzeichnet, dass** die Längskanäle (10, 11) eine Bodenwand und äußere, vertikale, parallele Längsseitenwände (2, 3) sowie eine gemeinsame Mittenwand (8) aufweisen, so dass sich jeweils ein rechteckiger Kanalquerschnitt ergibt und
dass die jeweils endseitig anschließenden Kanalumlenkbögen (13, 14) durch die Bodenwand und halbkreisförmig gebogene, äußere, vertikale Bogenwände (4, 5) gebildet sind.

5. Biogasanlage nach Anspruch 4, **dadurch gekennzeichnet, dass** die Längserstreckung eines Längskanals (10, 11) im Vergleich zu seiner Quererstreckung (12) wenigstens einmal, vorzugsweise wenigstens zweimal und besonders bevorzugt wenigstens 2,5-mal so groß ist.

6. Biogasanlage nach einem der Ansprüche 1 bis 5, **dadurch gekennzeichnet, dass** die Wände des Fermenterbehälters (1) aus Beton und/oder Stahl und/oder Kunststoff ggf. aus zusammenfügbaren Fertigteilelementen hergestellt sind.

7. Biogasanlage nach einem der Ansprüche 1 bis 6, **dadurch gekennzeichnet, dass** die Außenwände der Längskanäle (10, 11) in Querrichtung äußere Stützbauten (24, 25) aufweisen oder gegenüberliegende Außenwände im oberen Bereich durch Zugklammern, vorzugsweise in einer Doppelfunktion als begehbare und Technikelemente tragende Brückenstege (27) zusammengespannt sind.

8. Biogasanlage nach einem der Ansprüche 1 bis 7, **dadurch gekennzeichnet, dass** die Längskanäle (10, 11) und Kanalumlenkbögen (13, 14) durch eine Betondecke (23) und/oder durch Stahlplatten und/oder durch ein Foliendach (26) gasdicht abgedeckt sind.

9. Biogasanlage nach Anspruch 8, **dadurch gekennzeichnet, dass** die Längskanäle (10, 11) durch ein giebeldachförmiges Foliendach (26) und die halbkreisförmigen Kanalumlenkbögen (13, 14) jeweils durch eine Betondecke (23', 23") abgedeckt sind.

10. Biogasanlage nach Anspruch 8 oder 9, **dadurch gekennzeichnet, dass** in den Betondecken- und/oder Foliendachbereichen Revisionsöffnungen, insbesondere Revisionsschächte (17, 18) und/oder Sichtfenster (21, 22) angeordnet sind.

11. Biogasanlage nach einem der Ansprüche 1 bis 10, **dadurch gekennzeichnet, dass** vorzugsweise in den Längskanälen (10, 11) und/oder ggf. in den Kanalumlenkbögen (13, 14) wenigstens eine Rühreinrichtung (19, 20) angeordnet ist, mit der das Substrat im Fermenterbehälter (1) in eine in der Endlos-Kanalstromstruktur umlaufende Bewegung versetzbar ist.

12. Biogasanlage nach Anspruch 11, **dadurch gekennzeichnet, dass** die wenigstens eine Rühreinrichtung ein Tauchrührgerät (19, 20) ist, das einen elektrisch oder hydraulisch antriebbaren Motor aufweist und das vorzugsweise höhenverstellbar durch einen Revisionsschacht (17, 18) im Fermenterbehälter (1) oder an einem Brückensteg (27) angeordnet ist.

13. Biogasanlage nach einem der Ansprüche 1 bis 13, **dadurch gekennzeichnet, dass** wenigstens zwei Fermenterbehälter (1) und/oder ein Endlagerbehälter (30) mit gleichem Aufbau längsparallel nebeneinander angeordnet sind, ggf. längsparallel zu einer Silowand (28).

14. Biogasanlage nach Anspruch 13, **dadurch gekennzeichnet, dass** zumindest ein Fermenterbehälter (1) oder ein Endlagerbehälter (30) eine vergleichsweise geringere Längserstreckung aufweist und in dem dadurch geschaffenen Freiraum (31) ein Technikgebäude (32), insbesondere für Pumpen und/oder für Steuerungen und/oder für ein Blockheizkraftwerk (BHKW), oder ein Gasspeicherraum angeordnet sind.
